# EUROPEAN PATENT APPLICATION

(11) **EP 4 837 573 A1**
(43) Date of publication of application: **11.11.2026**
(21) Application number: 25742308.7
(22) Date of filing: 16.01.2025
(51) Int. Cl.: A61K 31/4184, A61K 31/351, A61K 45/06, A61P 3/04

(54) **PHARMACEUTICAL COMPOSITION CONTAINING SODIUM-GLUCOSE COTRANSPORTER-2 INHIBITOR AND ANGIOTENSIN II RECEPTOR BLOCKER FOR PREVENTION OR TREATMENT OF OBESITY**

(30) Priority: 18.01.2024 KR 20240007808
(71) Applicant: THPharm Corp., Cheongju-si, Chungcheongbuk-do 28160 (KR)
(72) Inventor: HAN, Tae Hee, Cheongju-si, Chungcheongbuk-do 28165 (KR); YOO, Yung Geun, Seoul 06256 (KR)
(74) Representative: Sander, Rolf
(86) International application number: PCT/KR2025/099042
(87) International publication number: WO 2025/155160

(57) **Abstract**

The present disclosure relates to a pharmaceutical composition for the prevention or treatment of obesity comprising a sodium-glucose cotransporter-2 inhibitor and an angiotensin II receptor blocker. The pharmaceutical composition is free from drug-drug interaction issues, exhibits a synergistic body weight-reducing effect, induces a rapid reduction in body weight during an early stage of administration, and maintains the reduced body weight independently of food intake during long-term administration. Accordingly, the pharmaceutical composition may be effectively applied to obese patients.

## Description

### [Technical Field]

The present disclosure relates to a pharmaceutical composition comprising a sodium-glucose cotransporter-2 inhibitor and an angiotensin II receptor blocker for the prevention or treatment of obesity.

### [Background Art]

The development of appetite-reducing drugs as an ultimate treatment for obesity has been a difficult process, as it attempts to reverse thousands of years of human evolution within only a few decades. The development of such drugs has involved considerable trial and error. Obesity is classified as a disease and has been reported to be associated with type 2 diabetes, cardiovascular disease, osteoarthritis, certain cancers, sleep apnea, asthma, and non-alcoholic fatty liver disease. The objective of pharmacological treatment of obesity is not merely weight loss, but, more importantly, improvement of obesity-associated comorbidities, such as hyperglycemia, dyslipidemia, and atherosclerotic heart disease. Anti-obesity drugs newly approved by the U.S. Food and Drug Administration (FDA) in recent years after extensive development efforts include lorcaserin, phentermine, topiramate, bupropion, naltrexone, and glucagon-like peptide-1 (GLP-1) receptor agonists.

However, the use of conventional anti-obesity agents may be associated with various adverse effects. For example, metformin may be associated with lactic acidosis or gastrointestinal adverse effects; sulfonylureas, glinides, and insulin or insulin analogues may be associated with hypoglycemia and weight gain; thiazolidinediones may be associated with edema, bone fractures, weight gain, and heart failure or other cardiac effects; and α-glucosidase inhibitors and GLP-1 or GLP-1 analogues may be associated with gastrointestinal adverse effects, such as dyspepsia, flatulence, diarrhea, nausea, or vomiting, and, most seriously, although rarely, pancreatitis. Therefore, there remains a need in the art to provide an effective, safe, and acceptable therapy, particularly for patients who are obese or overweight.

In addition, metabolic diseases are multifactorial in nature. Under certain circumstances, drugs having different mechanisms of action have been combined. However, an arbitrary combination of drugs having different modes of action does not necessarily result in a combination of their beneficial effects. Accordingly, there is a need for combination therapies that cause fewer adverse effects.

Accordingly, the present inventors conducted research to develop a combination therapy that can be effectively applied to the treatment of obesity, and thereby completed the present disclosure.

### [Disclosure]

### [Technical Problem]

One object of the present disclosure is to provide a pharmaceutical composition for the prevention or treatment of obesity, comprising an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient.

Another object of the present disclosure is to provide a method for preventing or treating obesity, comprising administering the pharmaceutical composition to a subject.

### [Technical Solution]

One aspect of the present disclosure provides a pharmaceutical composition for the prevention or treatment of obesity, comprising an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient.

According to an embodiment of the present disclosure, the sodium-glucose cotransporter-2 (SGLT-2) inhibitor may be dapagliflozin or empagliflozin.

According to an embodiment of the present disclosure, the angiotensin II receptor blocker may be telmisartan.

According to an embodiment of the present disclosure, the first pharmaceutical ingredient may be present in an amount of 40 mg to 80 mg, and the second pharmaceutical ingredient may be present in an amount of 10 mg.

### [Advantageous Effects]

According to the pharmaceutical composition for the prevention or treatment of obesity comprising a sodium-glucose cotransporter-2 inhibitor and an angiotensin II receptor blocker, the composition is free from drug-drug interaction issues, exhibits a synergistic effect on weight loss, induces rapid weight loss during an early stage of administration, and maintains the reduced body weight independently of food intake during long-term administration. Accordingly, the pharmaceutical composition may be effectively applied to obese patients.

### [Description of Drawings]

FIG. 1 is a diagram showing results of evaluating body weight loss and heart weight loss effects in male rats (A) and female rats (B) administered a combination of dapagliflozin and telmisartan at 1/8 mg/kg/day (low-dose group, G2), 3/24 mg/kg/day (medium-dose group, G3), or 9/72 mg/kg/day (high-dose group, G4), a dapagliflozin-alone administration group administered 9 mg/kg/day of dapagliflozin (G5), a telmisartan-alone administration group administered 72 mg/kg/day of telmisartan (G6), and a control group administered a 0.5% MC aqueous solution (G1).
FIG. 2 is a diagram showing a body weight-reducing effect in male rats following nonclinical 13-week repeated administration of dapagliflozin and/or telmisartan.
FIG. 3 is a diagram showing a body weight-reducing effect in female rats following nonclinical 13-week repeated administration of dapagliflozin and/or telmisartan.
FIG. 4 is a diagram showing food intake in male rats following nonclinical 13-week repeated administration of dapagliflozin and/or telmisartan.
FIG. 5 is a graph showing a body weight-reducing effect in a rat model following nonclinical 13-week repeated administration of dapagliflozin and/or telmisartan.
FIG. 6 is a graph showing a body weight-reducing effect resulting from administration of a combination of dapagliflozin and telmisartan in a high-fat diet-induced obese mouse model.
FIG. 7 is a graph showing a food intake reduction effect resulting from administration of a combination of dapagliflozin and telmisartan in a high-fat diet-induced obese mouse model.
FIG. 8 is a graph showing improvement effects on AST and ALT, which are blood markers of liver injury, and triglyceride (TG), which is a blood lipid marker, resulting from administration of a combination of dapagliflozin and telmisartan in a high-fat diet-induced obese mouse model.
FIG. 9 is a graph showing epididymal white adipose tissue weight (Epididymal-WAT weight) and retroperitoneal white adipose tissue weight (Retro-WAT weight) following administration of a combination of dapagliflozin and telmisartan in a high-fat diet-induced obese mouse model.
FIG. 10 is a graph showing white adipose tissue weight and total adipose tissue weight following administration of a combination of dapagliflozin and telmisartan in a high-fat diet-induced obese mouse model.
FIG. 11 is a dual-energy X-ray absorptiometry (DEXA) scan image showing an abdominal fat reduction effect in a combination high-dose group (T07), as compared with an obese group (T02), following administration of a combination of dapagliflozin and telmisartan in a high-fat diet-induced obese mouse model.

### [Best Mode]

One aspect of the present disclosure provides a pharmaceutical composition for the prevention or treatment of obesity, comprising an angiotensin II receptor blocker or a pharmaceutically acceptable salt thereof as a first pharmaceutical ingredient; and a sodium-glucose cotransporter-2 (SGLT-2) inhibitor or a pharmaceutically acceptable salt thereof as a second pharmaceutical ingredient.

In the present disclosure, it was confirmed that a combination of a sodium-glucose cotransporter-2 (SGLT-2) inhibitor and an angiotensin II receptor blocker (ARB), which have conventionally been administered separately, exhibits a synergistic effect in the treatment of obesity. In addition, a drug-drug compatibility test was performed to derive an optimal combination of the SGLT-2 inhibitor and the ARB, and it was confirmed that the pharmaceutical composition according to an embodiment of the present disclosure may be effectively applied to the prevention or treatment of obesity.

As used herein, the term "overweight" may be defined as a condition in which an individual has a body mass index (BMI) of 25 kg/m² or more and less than 30 kg/m². The terms "overweight" and "pre-obese" are used interchangeably herein.

The term "obesity" may be defined as a condition in which an individual has a body mass index (BMI) of 30 kg/m² or more. According to the World Health Organization (WHO) definition, obesity may be classified as follows: class I obesity is a condition in which the body mass index (BMI) is 30 kg/m² or more and less than 35 kg/m²; class II obesity is a condition in which the body mass index (BMI) is 35 kg/m² or more and less than 40 kg/m²; and class III obesity is a condition in which the body mass index (BMI) is 40 kg/m² or more. Obesity may include, for example, visceral obesity or abdominal obesity, and may include obese patients who do not suffer from diabetes, particularly type 1 or type 2 diabetes.

Visceral obesity may be defined as a condition in which a waist-to-hip ratio is measured as 1.0 or more in men and 0.8 or more in women. The waist-to-hip ratio may be used to define a risk of developing insulin resistance and prediabetes.

Abdominal obesity may generally be defined as a condition in which the waist circumference exceeds 40 inches or 102 cm in men and exceeds 35 inches or 94 cm in women. For Japanese individuals or Japanese patients, abdominal obesity may be defined as a condition in which the waist circumference is 85 cm or more in men and 90 cm or more in women (see, for example, the Japanese Committee for the Diagnostic Criteria of Metabolic Syndrome).

The present disclosure relates to a method for reducing and maintaining body weight and/or body fat in a patient in need thereof, for example, an overweight or obese patient, the method comprising administering a sodium-glucose cotransporter-2 (SGLT-2) inhibitor and an angiotensin II receptor blocker.

The SGLT-2 inhibitor included in the pharmaceutical composition of the present disclosure may be any one substance selected from the group consisting of dapagliflozin, empagliflozin, ipragliflozin, canagliflozin, luseogliflozin, and tofogliflozin. Dapagliflozin or empagliflozin is preferable in terms of combination compatibility and synergistic effect, and dapagliflozin is most preferable.

The composition of the present disclosure may comprise a pharmaceutically acceptable carrier. The pharmaceutically acceptable carrier included in the composition of the present disclosure may be one generally used in the preparation of pharmaceutical products, and examples thereof include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil. In addition to the above ingredients, the pharmaceutical composition of the present disclosure may further comprise a lubricant, a wetting agent, a sweetening agent, a flavoring agent, an emulsifying agent, a suspending agent, a preservative, and the like. Suitable pharmaceutically acceptable carriers and preparations are described in detail in Remington: The Science and Practice of Pharmacy, 22nd Edition (2013).

The pharmaceutical composition according to an embodiment of the present disclosure may be administered together with one or more substances exhibiting pharmacological activity for the prevention or treatment of obesity.

In addition, the pharmaceutical composition according to an embodiment of the present disclosure may be used alone or in combination with procedures, surgery, hormone therapy, drug therapy, and/or methods using biological response modifiers for the prevention or treatment of obesity.

The composition of the present disclosure may comprise various bases and/or additives necessary and suitable for preparing a formulation thereof. The composition may be prepared by further comprising known compounds, within a range that does not reduce the effect thereof, such as nonionic surfactants, silicone polymers, extenders, fragrances, preservatives, disinfectants, oxidation stabilizers, organic solvents, ionic or nonionic thickeners, softeners, antioxidants, free-radical scavengers, opacifying agents, stabilizers, emollients, silicones, α-hydroxy acids, antifoaming agents, moisturizers, vitamins, insect repellents, flavoring agents, preservatives, surfactants, anti-inflammatory agents, substance P antagonists, fillers, polymers, propellants, alkalizing or acidifying agents, or coloring agents.

A suitable dose of the composition of the present disclosure may be variously prescribed depending on factors such as a formulation method, a mode of administration, the age, weight, sex, and pathological condition of a patient, food intake, an administration time, an administration route, an excretion rate, and response susceptibility. The dose of the composition of the present disclosure may be 0.001 to 1,000 mg/kg for an adult.

The pharmaceutical composition of the present disclosure may be prepared by mixing the angiotensin II receptor blocker as the first pharmaceutical ingredient with the SGLT-2 inhibitor as the second pharmaceutical ingredient. The mixing may be performed simultaneously or sequentially and may be performed using methods known in the art.

The composition of the present disclosure may be orally administered.

When orally administered, the composition of the present disclosure may be administered in various dosage forms, including tablets, pills, hard or soft capsules, liquids, suspensions, emulsions, syrups, granules, elixirs, and troches, and may further comprise various excipients such as wetting agents, sweetening agents, flavoring agents, and preservatives.

Specifically, when the composition of the present disclosure is formulated into an oral dosage form, the composition may further comprise suitable carriers, excipients, and diluents commonly used in the preparation thereof. Examples of the carriers, excipients, and diluents include, but are not limited to, lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, and/or mineral oil.

In addition, the composition may be formulated to comprise diluents or excipients commonly used in formulation, such as fillers, extenders, binders, wetting agents, disintegrants, and surfactants, and may further comprise a lubricant such as magnesium stearate or talc in addition to the excipients.

The composition of the present disclosure may comprise about 0.1% to about 90%, preferably about 1% to about 80%, of the pharmaceutical ingredients. A pharmaceutical preparation for oral use may be obtained by mixing the active ingredients with a solid excipient, preferably granulating the resulting mixture, and, if necessary or desirable, processing the mixture or granules into tablets or coated tablet cores after adding suitable auxiliary substances.

The pharmaceutical composition of the present disclosure may be, for example, a bilayer tablet comprising an angiotensin II receptor blocker as the first pharmaceutical ingredient and an SGLT-2 inhibitor as the second pharmaceutical ingredient. Alternatively, the pharmaceutical composition may have an inner-core tablet structure comprising telmisartan as the first pharmaceutical ingredient, an SGLT-2 inhibitor as the second pharmaceutical ingredient, and an outer layer surrounding an inner core layer, but is not limited thereto.

According to an embodiment of the present disclosure, the sodium-glucose cotransporter-2 (SGLT-2) inhibitor may be dapagliflozin or empagliflozin.

As used herein, the term "dapagliflozin" refers to (1S)-1,5-anhydro-1-C-{4-chloro-3-[(4-ethoxyphenyl)methyl]phenyl}-D-glucitol, and the term "empagliflozin" refers to (1S)-1,5-anhydro-1-C-[4-chloro-3-[[4-[[(3S)-tetrahydro-3-furanyl]oxy]phenyl]methyl]phenyl]-D-glucitol.

According to an embodiment of the present disclosure, the angiotensin II receptor blocker may be telmisartan.

As used herein, the term "telmisartan" refers to 4'-[(1,4'-dimethyl-2'-propyl[2,6'-bi-1H-benzimidazol]-1'-yl)methyl][1,1'-biphenyl]-2-carboxylic acid.

In the present disclosure, it was confirmed that, among ARBs, telmisartan exhibits superior combination compatibility and formulation stability with SGLT-2 inhibitors, particularly dapagliflozin and empagliflozin, as compared with olmesartan and valsartan.

Dapagliflozin, empagliflozin, or telmisartan may be understood to include active metabolites and prodrugs thereof. The term "metabolite" refers to each active derivative that may be produced when dapagliflozin, empagliflozin, or telmisartan is metabolized. The term "prodrug" refers to a compound that is metabolized into dapagliflozin, empagliflozin, or telmisartan, or into the same metabolite or metabolites as dapagliflozin, empagliflozin, or telmisartan. In addition, dapagliflozin, empagliflozin, or telmisartan may include pharmaceutically acceptable salts thereof, crystalline forms thereof, hydrates, solvates, diastereomers, or enantiomers.

According to an embodiment of the present disclosure, the first pharmaceutical ingredient may be present in an amount of 40 mg to 80 mg, and the second pharmaceutical ingredient may be present in an amount of 10 mg.

When the first pharmaceutical ingredient is included in an amount of less than 40 mg and/or the second pharmaceutical ingredient is included in an amount of less than 10 mg, an appropriate therapeutic effect for diabetes and hypertension may not be expected due to a low blood concentration of the drug and the resulting low pharmacological effect.

On the other hand, when the first pharmaceutical ingredient is included in an amount of more than 80 mg and/or the second pharmaceutical ingredient is included in an amount of more than 10 mg, problems such as a high blood concentration of the drug, resulting adverse effects, manifestation of toxicity, and induction of hypoglycemia or hypotension due to excessive therapeutic effects may occur, and an appropriate therapeutic effect may not be expected.

In an embodiment, the tablet may comprise 40 mg and 10 mg, or 80 mg and 10 mg, of the first pharmaceutical ingredient and the second pharmaceutical ingredient, respectively.

Another aspect of the present disclosure provides a method for preventing or treating obesity, comprising administering the pharmaceutical composition to a subject.

The angiotensin II receptor blocker or a pharmaceutically acceptable salt thereof, and the SGLT-2 inhibitor or a pharmaceutically acceptable salt thereof, included in the pharmaceutical composition of the present disclosure may be orally administered to a subject or patient in an amount effective for treatment or prevention, depending on the intended purpose.

It should be understood that the dose administered to a particular subject or patient should be determined based on various relevant factors, including the patient's weight, age, race, sex, health condition, diet, administration time, administration method, and severity of disease, and may be appropriately adjusted by a medical practitioner.

For example, a physician may initiate administration of the pharmaceutical composition of the present disclosure at a dose lower than that required to achieve a desired therapeutic effect and gradually increase the dose until the desired effect is achieved, and may readily determine and prescribe an appropriate dose as needed.

### [Modes of the Invention]

Hereinafter, the present disclosure will be described in more detail with reference to Examples. However, these Examples are provided merely to illustrate the present disclosure, and the scope of the present disclosure is not limited thereto.

### Experimental Example 1. Confirmation of Heart Weight Reduction and Promotion of Urinary Excretion by a Combination of an ARB and an SGLT-2 Inhibitor

### 1-1. Nonclinical 13-Week Repeated Administration Method

The heart weight-reducing effect and urinary excretion-promoting effect of a combination of telmisartan, an angiotensin II receptor blocker (ARB), and dapagliflozin, a sodium-glucose cotransporter-2 (SGLT-2) inhibitor, were evaluated.

Specifically, the groups were configured as shown in Table 1. Ten male and ten female 6-week-old Sprague-Dawley rats per group were orally administered the test substances once daily for 13 weeks. During the test period, general clinical signs were observed, blood tests were performed, and urinary excretion volume was measured. After completion of the observation period, the rats were euthanized and necropsied, and the heart weight was measured.

**[Table 1]**

| **Group** | **Classification** | **Dose (mg/kg/day) Dapagliflozin** | **Dose (mg/kg/day) Telmisartan** | **Dose Volume (mL/kg)** |
|---|---|---|---|---|
| G1 | Normal control group | 0 | 0 | 10 |
| G2 | Combination low-dose group | 1 | 8 | 10 |
| G3 | Combination medium-dose group | 3 | 24 | 10 |
| G4 | Combination high-dose group | 9 | 72 | 10 |
| G5 | Dapagliflozin-alone administration group | 9 | 0 | 10 |
| G6 | Telmisartan-alone administration group | 0 | 72 | 10 |

Dapagliflozin propanediol hydrate was used as dapagliflozin, and telmisartan powder (23.9%) was used as telmisartan, each in an amount corresponding to the respective dose.

### 1-2. Confirmation of Heart Weight Reduction and Promotion of Urinary Excretion by the Combination of Dapagliflozin and Telmisartan

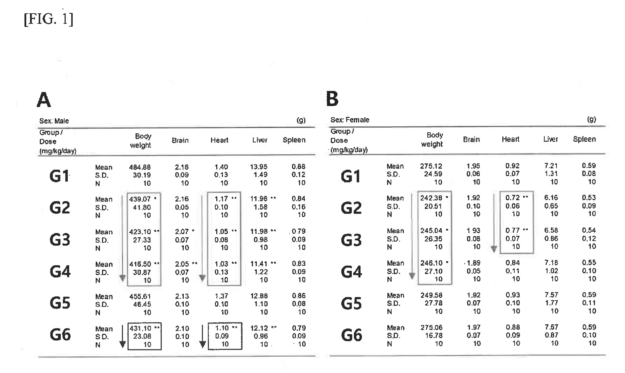

As a result of repeated oral administration of dapagliflozin and/or telmisartan, alone or in combination, to the rats for 13 weeks in Experimental Example 1-1, a significant reduction in heart weight was observed in the low-dose, medium-dose, and high-dose groups administered the combination of dapagliflozin and telmisartan. In particular, in female rats, a significant reduction in heart weight was observed in the low-dose and medium-dose groups administered the combination of dapagliflozin and telmisartan (FIG. 1).

In addition, increased urinary excretion was observed in the low-dose, medium-dose, and high-dose groups administered the combination of dapagliflozin and telmisartan.

These results confirmed that cardiac hypertrophy caused by obesity may be reduced by the combination of dapagliflozin and telmisartan.

### Experimental Example 2. Confirmation of a Body Weight-Reducing Effect Independent of Food Intake by a Combination of an ARB and an SGLT-2 Inhibitor

### 2-1. Confirmation of the Body Weight-Reducing Effect of the Combination of Dapagliflozin and Telmisartan

Dapagliflozin and/or telmisartan, alone or in combination, were repeatedly administered orally to normal SD rats for 13 weeks in the same manner as in Experimental Example 1-1, and body weight was measured.

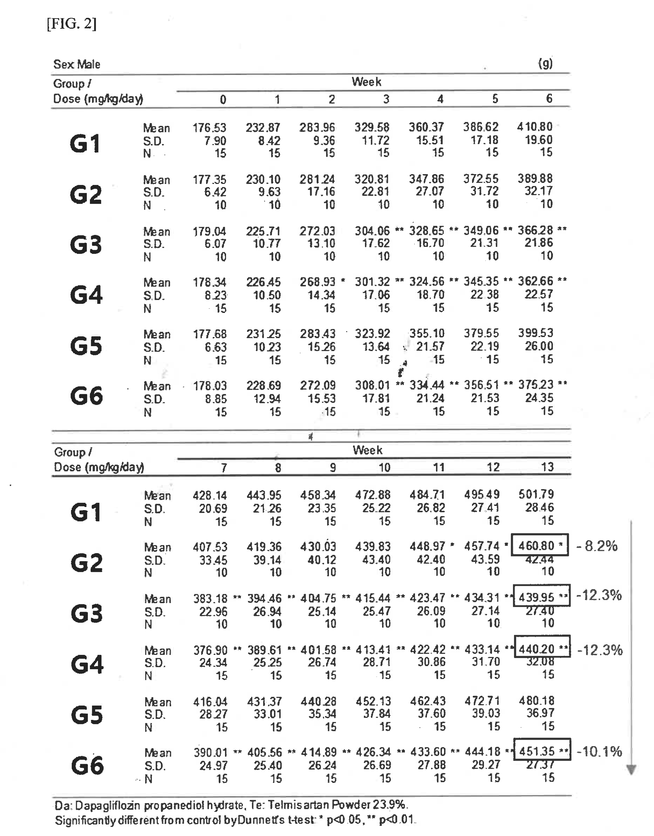

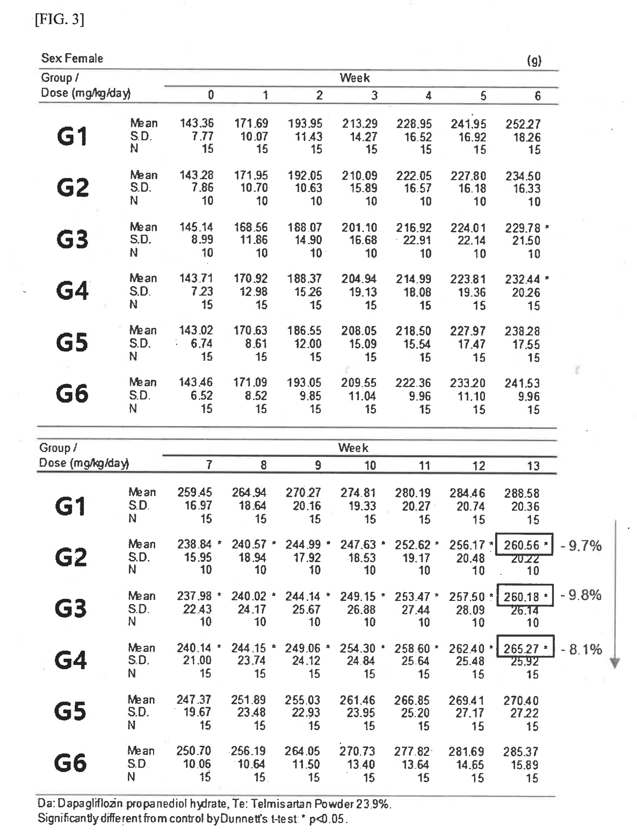

As a result, a significant reduction in body weight was observed in the low-dose, medium-dose, and high-dose groups administered the combination of dapagliflozin and telmisartan. In contrast, no significant reduction in body weight was observed in female rats in the telmisartan-alone administration group, and no significant reduction in body weight was observed in either female or male rats in the dapagliflozin-alone administration group (FIGS. 2 and 3).

These results confirmed a synergistic effect of the combination of dapagliflozin and telmisartan on body weight loss.

### 2-2. Confirmation of the Body Weight-Reducing Effect of the Combination of Dapagliflozin and Telmisartan Independent of Food Intake

Dapagliflozin and/or telmisartan, alone or in combination, were repeatedly administered orally to normal SD rats for 13 weeks in the same manner as in Experimental Example 1-1. Food intake was measured, and its relationship with body weight was analyzed.

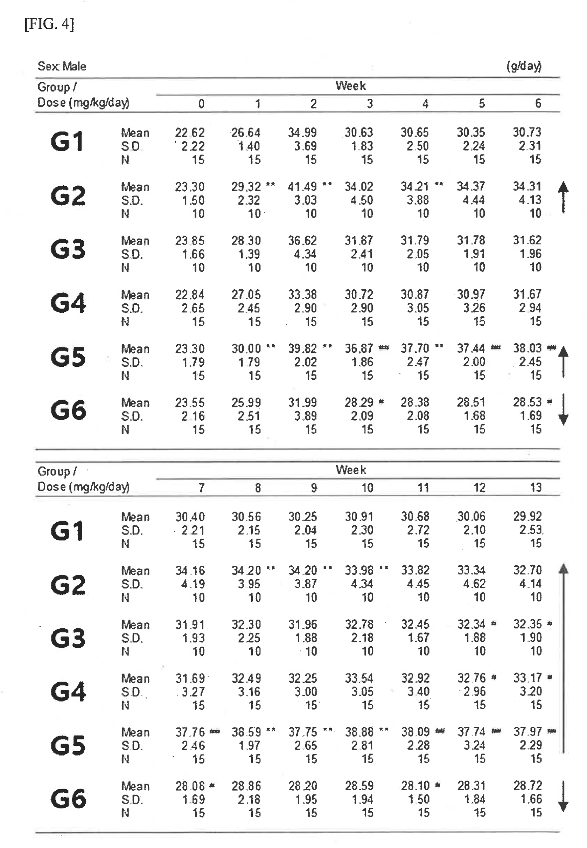

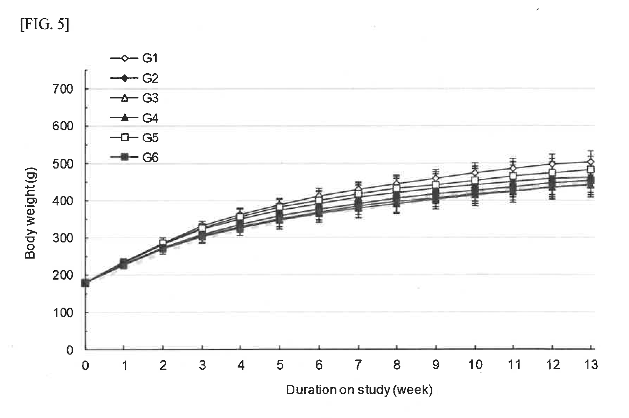

As a result, food intake was significantly increased in the low-dose, medium-dose, and high-dose groups administered the combination of dapagliflozin and telmisartan (FIG. 4), while a body weight reduction of 12.3% was observed in the medium-dose group administered the combination of dapagliflozin and telmisartan (FIG. 5).

These results confirmed that the combination of dapagliflozin and telmisartan exhibited a body weight-reducing effect in normal rats in which obesity had not been induced, despite an increase in food intake, and thus may effectively reduce body weight independently of food intake.

### Experimental Example 3. Confirmation of the Obesity-Ameliorating Effect of a Combination of an ARB and an SGLT-2 Inhibitor in a High-Fat Diet-Induced Obese Mouse Model

### 3-1. Establishment of a High-Fat Diet-Induced Obese Mouse Model and Design of Experimental Groups

To evaluate the obesity-ameliorating effect of a combination of telmisartan, an angiotensin II receptor blocker, and dapagliflozin, a sodium-glucose cotransporter-2 inhibitor, a high-fat diet-induced obese mouse model was established, and the experimental groups were designed.

Specifically, the experimental groups for evaluating the obesity-ameliorating effect were configured as shown in Table 2. The normal control group was prepared by feeding male C57BL/6J mice a standard chow diet for 4 weeks. The obese mouse model was prepared by feeding male C57BL/6J mice a 60% kcal high-fat diet (HFD) for 4 weeks and then dividing the mice into an obese group and an experimental group such that the groups had similar mean body weights. Thereafter, the test substances were administered for 5 weeks while each group was fed either the standard chow diet or the HFD.

**[Table 2]**

| **Group** | **Test Substance** | **Dose Volume** |
|---|---|---|
| T01 | Normal control group (n=8), distilled water | 10 mL/kg |
| T02 | Obese group (n=8), distilled water | 10 mL/kg |
| T07 | Combination high-dose group (n=8), dapagliflozin 9 mg/kg/day + telmisartan 72 mg/kg/day | 10 mL/kg |

Dapagliflozin propanediol hydrate was used as dapagliflozin, and telmisartan powder (23.9%) was used as telmisartan, each in an amount corresponding to the respective dose.

During the 9-week experimental period, body weight and food intake were measured twice weekly. Upon completion of the 9-week experiment, whole-body images of the mice were obtained using a dual-energy X-ray absorptiometry (DEXA) scanner, blood was collected, and the mice were dissected for use in subsequent experiments.

### 3-2. Confirmation of the Body Weight-Reducing Effect

As a result of measuring the body weight of each experimental group according to Experimental Example 3-1, the body weight of the obese group was significantly increased compared with that of the normal control group, whereas the body weight of the combination high-dose group was significantly decreased compared with that of the obese group.

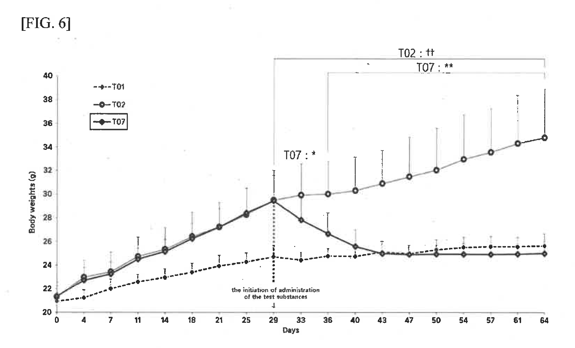

In particular, the body weight of the combination high-dose group began to decrease significantly compared with that of the obese group as early as 4 days after initiation of administration of the test substances. Two weeks after initiation of administration of the test substances, the body weight of the combination high-dose group was statistically significantly reduced to a level comparable to that of the normal control group. A body weight reduction of 28.2% was observed in the combination high-dose group compared with the obese group (FIG. 6).

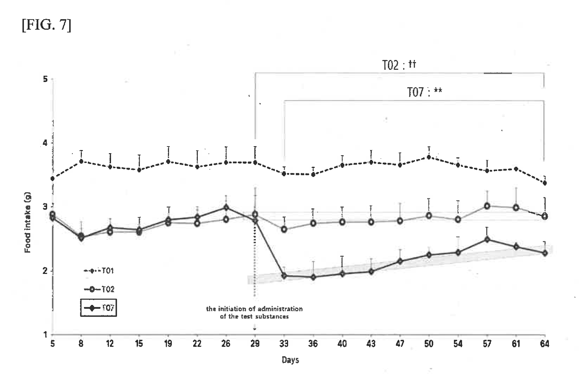

In addition, as a result of measuring the food intake of each experimental group according to Experimental Example 3-1, the food intake of the combination high-dose group was significantly decreased compared with that of the obese group. At the end of the experiment, the food intake of the combination high-dose group was reduced by 21.4% compared with that of the obese group (FIG. 7).

Meanwhile, as a result of follow-up observation of body weight and food intake in each experimental group for 3 weeks after completion of the experiment, the food intake of the combination high-dose group (2.90 g/day) recovered to a level similar to that of the obese group (3.08 g/day), whereas the body weight of the combination high-dose group remained reduced by 25.5% compared with that of the obese group.

These results confirmed that the combination of dapagliflozin and telmisartan induces reductions in food intake and body weight during an early stage of administration in obese mice and, during long-term administration, effectively maintains the reduced body weight despite food intake returning to a level similar to that before administration. Accordingly, the combination may provide a long-term effect of maintaining reduced body weight in obese patients independently of food intake.

### 3-3. Confirmation of Improvement in Blood Biochemical Parameters

The blood obtained in Experimental Example 3-1 was collected in SST tubes (BD, USA) and centrifuged at 10,000 rpm for 15 minutes using a centrifuge (LABOGENE, USA). The serum was separated, aliquoted, and stored at -80°C.

Thereafter, the levels of aspartate aminotransferase (AST) and alanine aminotransferase (ALT), which are blood markers of liver injury, and triglyceride (TG), which is a blood lipid marker, were measured in the stored serum using a HITACHI 7180 analyzer (Japan).

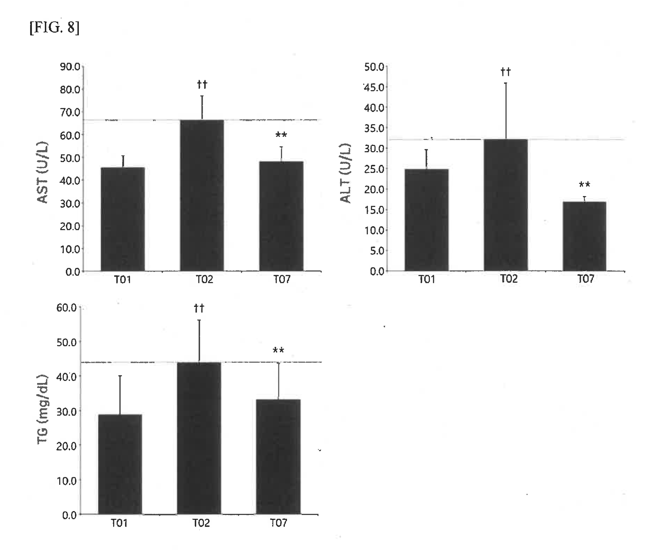

As a result, both the liver injury markers and the blood lipid marker were significantly increased in the obese group compared with the normal control group. In contrast, AST, ALT, and TG were all statistically significantly decreased in the combination high-dose group compared with the obese group (FIG. 8). In particular, TG was reduced by 51.8%.

These results confirmed that the combination of dapagliflozin and telmisartan may improve blood markers of liver injury and blood lipid markers without causing adverse effects on the liver, kidneys, or muscles.

### 3-4. Confirmation of Fat Reduction Effect

The weights of white adipose tissue (WAT) and brown adipose tissue (BAT) in the epididymal adipose tissue and retroperitoneal adipose tissue obtained by dissection in Experimental Example 3-1 were measured. Based thereon, epididymal white adipose tissue weight (Epididymal-WAT weight), retroperitoneal white adipose tissue weight (Retro-WAT weight), white adipose tissue weight, and total adipose tissue weight were determined.

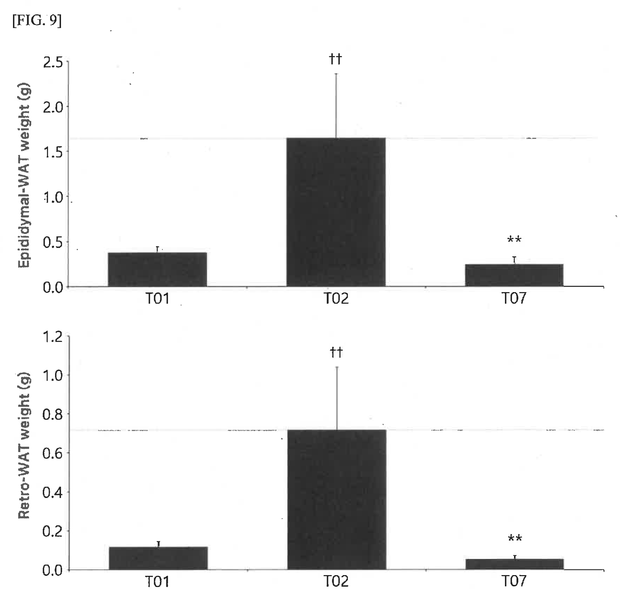

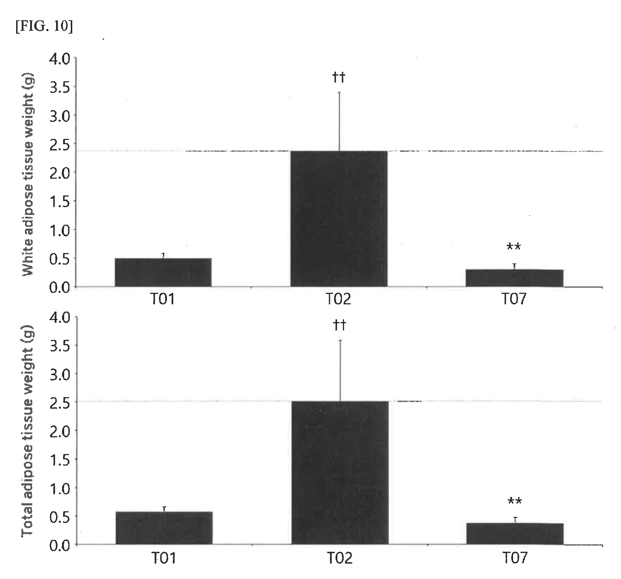

As a result, the weight of each adipose tissue was significantly increased in the obese group compared with the normal control group. In contrast, the weight of each adipose tissue was significantly decreased in the combination high-dose group compared with the obese group (FIGS. 9 and 10).

The epididymal white adipose tissue weight, retroperitoneal white adipose tissue weight, white adipose tissue weight, and total adipose tissue weight in the combination high-dose group were reduced by 85.1%, 92.6%, 87.3%, and 85.2%, respectively, compared with those in the obese group.

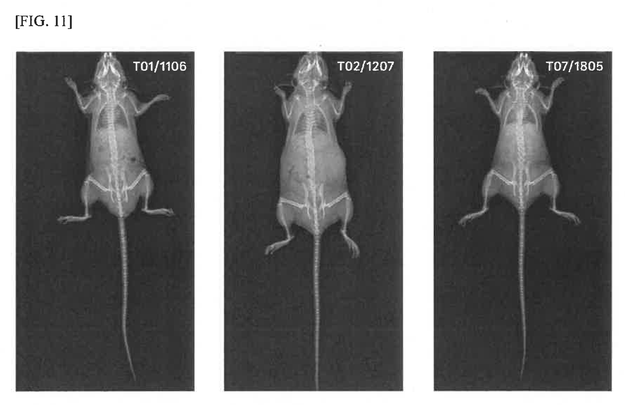

In addition, comparison of the DEXA scan images obtained in Experimental Example 3-1 confirmed that the abdominal fat of the combination high-dose group was reduced to a level comparable to that of the normal control group (FIG. 11).

### 3-5. Statistical Analysis

The results of Experimental Example 3 are expressed as the mean ± standard deviation (SD). All experimental results were analyzed using an independent-samples t-test.

Significant differences between the normal control group (T01) and the obese group (T02), as determined by the independent-samples t-test, are indicated by †† for p<0.01 and † for p<0.05. Significant differences between the obese group (T02) and the combination high-dose group (T07), as determined by the independent-samples t-test, are indicated by ** for p<0.01 and * for p<0.05.

### Experimental Example 4. Identification of an Optimal Combination of an ARB and an SGLT-2 Inhibitor

A drug-drug compatibility test was conducted to identify an optimal combination of an angiotensin II receptor blocker (ARB) and a sodium-glucose cotransporter-2 (SGLT-2) inhibitor that remains stable without degradation during long-term storage.

Specifically, mixtures of an ARB and an SGLT-2 inhibitor were stored for 2 weeks under accelerated stability test conditions of 40±2°C and 75±5% relative humidity, and changes in the contents of the respective ingredients and related substances, as well as changes in appearance, such as color and agglomeration, were analyzed. Olmesartan, valsartan, and telmisartan were used as the ARBs, and empagliflozin, dapagliflozin propanediol monohydrate, and canagliflozin were used as the SGLT-2 inhibitors. The amounts of the respective ingredients were configured as shown in Table 3.

**[Table 3]**

| **Classification** | **ARB** | **SGLT-2 Inhibitor** |
|---|---|---|
| Example 1 | Telmisartan 40 mg | Empagliflozin 40 mg |
| Example 2 | Telmisartan 40 mg | Dapagliflozin 40 mg |
| Comparative Example 1 | Telmisartan 80 mg | - |
| Comparative Example 2 | - | Empagliflozin 80 mg |
| Comparative Example 3 | - | Dapagliflozin 80 mg |
| Comparative Example 4 | Olmesartan 80 mg | - |
| Comparative Example 5 | Valsartan 80 mg | - |
| Comparative Example 6 | - | Canagliflozin 80 mg |
| Comparative Example 7 | Telmisartan 40 mg | Canagliflozin 40 mg |
| Comparative Example 8 | Olmesartan 40 mg | Empagliflozin 40 mg |
| Comparative Example 9 | Olmesartan 40 mg | Dapagliflozin 40 mg |
| Comparative Example 10 | Valsartan 40 mg | Empagliflozin 40 mg |
| Comparative Example 11 | Valsartan 40 mg | Dapagliflozin 40 mg |

To analyze the contents of the active ingredients and the degree of formation of related substances, ten tablets of each mixture of the ARB and the SGLT-2 inhibitor stored for 1 week or 2 weeks were placed in each of three 100 mL flasks. A solution prepared by mixing a 0.01 M aqueous hydrochloric acid solution and acetonitrile at an appropriate ratio was added to extract the active ingredients, followed by stirring and HPLC analysis.

To evaluate appearance and viscosity, 10 g of each mixture of the ARB and the SGLT-2 inhibitor stored for 1 week or 2 weeks was exposed to 100 mL of purified water at room temperature for 10 minutes, and the appearance and viscosity of the mixture were then evaluated.

As a result, at week 2 of the accelerated stability test, all evaluation parameters were found to be unacceptable in Comparative Examples 22 to 26 among the mixtures of the ARBs and the SGLT-2 inhibitors. In contrast, the contents of telmisartan and empagliflozin in Example 1 were 99.4% and 95.1%, respectively, and the contents of telmisartan and dapagliflozin in Example 2 were 99.2% and 99.2%, respectively, with the changes in content being within 10%. In addition, the total amounts of related substances were also found to be acceptable. Accordingly, these combinations were confirmed to exhibit the best drug-drug compatibility (Table 4).

**[Table 4]**

| **Classification** | **Test Item** | **Initial** | **Accelerated, Week 1** | **Accelerated, Week 2** |
|---|---|---|---|---|
| Example 1 | Content | 99.9/100 | 99.5/97.6 | 99.4/95.1 |
| | Related substances | 0.1 or less | 1.0 or more | 1.0 or more |
| | Appearance | Acceptable | Acceptable | Acceptable |
| Example 2 | Content | 99.9/100.1 | 99.4/99.9 | 99.2/99.2 |
| | Related substances | 0.1 or less | 0.1 or more | 0.1 or more |
| | Appearance | Acceptable | Acceptable | Acceptable |
| Comparative Example 1 | Content | 100.1 | 100.1 | 100 |
| | Related substances | 0.1 or less | 0.1 or less | 0.1 or less |
| | Appearance | Acceptable | Acceptable | Acceptable |
| Comparative Example 2 | Content | 99.9 | 97.3 | 95.1 |
| | Related substances | 0.1 or less | 1.0 or less | 1.0 or more |
| | Appearance | Acceptable | Acceptable | Acceptable |
| Comparative Example 3 | Content | 100.3 | 100.1 | 100.2 |
| | Related substances | 0.1 or less | 0.1 or less | 0.1 or less |
| | Appearance | Acceptable | Acceptable | Acceptable |
| Comparative Example 4 | Content | 99.9 | 99.7 | 99.7 |
| | Related substances | 0.1 or less | 0.1 or more | 0.1 or more |
| | Appearance | Acceptable | Acceptable | Acceptable |
| Comparative Example 5 | Content | 99.7 | 99.4 | 99.2 |
| | Related substances | 0.1 or less | 0.1 or more | 0.1 or more |
| | Appearance | Acceptable | Acceptable | Acceptable |
| Comparative Example 6 | Content | 99.5 | 95.2 | 89.2 |
| | Related substances | 0.1 or less | 1.0 or less | 5.0 or more |
| | Appearance | Acceptable | Unacceptable | Unacceptable |
| Comparative Example 7 | Content | 99.5/92.1 | 99.1/89.1 | 98.9/82.3 |
| | Related substances | 0.1 or less | 5.0 or more | 5.0 or more |
| | Appearance | Acceptable | Unacceptable | Unacceptable |
| Comparative Example 8 | Content | 100.1/99.9 | 99.1/90 | 95.4/84.6 |
| | Related substances | 0.1 or less | 5.0 or more | 5.0 or more |
| | Appearance | Acceptable | Acceptable | Unacceptable |
| Comparative Example 9 | Content | 100.1/100 | 97.8/89.1 | 95/79.7 |
| | Related substances | 0.1 or less | 5.0 or more | 5.0 or more |
| | Appearance | Acceptable | Acceptable | Unacceptable |
| Comparative Example 10 | Content | 100.2/99.9 | 95.5/95.4 | 93.3/90.1 |
| | Related substances | 0.1 or less | Acceptable | 5.0 or more |
| | Appearance | Acceptable | Unacceptable | Unacceptable |
| Comparative Example 11 | Content | 100.3/99.7 | 95.5/95.7 | 91.1/92.3 |
| | Related substances | 0.1 or less | 1.0 or more | 1.0 or more |
| | Appearance | Acceptable | Unacceptable | Unacceptable |

The present disclosure has been described above mainly with reference to the Examples thereof. Those skilled in the art will appreciate that the present disclosure may be embodied in various modified forms without departing from the essential characteristics thereof. Accordingly, the disclosed Examples should be considered in an illustrative rather than a restrictive sense. The scope of the present disclosure is defined by the appended claims rather than by the foregoing description, and all modifications and variations falling within the scope of the claims and equivalents thereof should be construed as being included within the present disclosure.

## Claims

1. A pharmaceutical composition for the prevention or treatment of obesity, comprising:
an angiotensin II receptor blocker or a pharmaceutically acceptable salt thereof as a first pharmaceutical ingredient; and
a sodium-glucose cotransporter-2 (SGLT-2) inhibitor or a pharmaceutically acceptable salt thereof as a second pharmaceutical ingredient.

2. The pharmaceutical composition for the prevention or treatment of obesity of claim 1, wherein the sodium-glucose cotransporter-2 (SGLT-2) inhibitor is dapagliflozin or empagliflozin.

3. The pharmaceutical composition for the prevention or treatment of obesity of claim 1, wherein the angiotensin II receptor blocker is telmisartan.

4. The pharmaceutical composition for the prevention or treatment of obesity of claim 1, wherein:
the first pharmaceutical ingredient is present in an amount of 40 mg to 80 mg; and
the second pharmaceutical ingredient is present in an amount of 10 mg.
